# FASCICULE DE BREVET EUROPEEN

(11) **EP 0 485 678 B1**
(45) Date de publication et mention de la délivrance du brevet: **12.02.1997**
(21) Numéro de dépôt: 90440103.1
(22) Date de dépôt: 16.11.1990
(51) Int. Cl.: A61F 2/32, A61F 2/34

(54) **Prothèse totale de hanche**
Hüftgelenkprothese
Hip joint prosthesis

(43) Date de publication de la demande: 20.05.1992
(73) Titulaire: SOCIETE CIVILE ESSOR, 74550 Perrignier (FR)
(72) Inventeur: Tarquini, Cyril, F-26740 Sauzet (FR); Trillaud, Jean-Marie, F-74540 Perrignier (FR); Granger, André, F-69250 St Germain au Mont Dore (FR); Augagneur, Christian, F-69126 Brindas (FR)
(74) Mandataire: Polus, Camille

(56) Documents cités:
- EP-A- 0 313 762
- DE-A- 2 911 754
- FR-A- 2 583 286
- FR-A- 2 598 609
- FR-A- 2 638 962
- US-A- 4 695 282
- US-A- 4 840 632

## Description

La présente invention est relative à une prothèse chirurgicale totale du genre de celles qu'on implante dans la hanche d'un patient en remplacement de l'articulation naturelle. Une telle prothèse totale comprend, d'une part une partie fémorale, d'autre part un cotyle. Une prothèse totale de la hanche selon les caractéristiques du préambule de la revendication 1 est connue du document FR-A-2 598 609.

Il existe de nombreux types de prothèses totales de la hanche, mais la plupart présentent divers inconvénients.

En particulier, il arrive qu'à la longue la partie fémorale se désolidarise plus ou moins de l'os du fémur, ou amorce un basculement à l'intérieur de celui-ci, généralement en varus. En ce qui concerne le cotyle, sa fixation et son immobilisation dans l'os du bassin laissent souvent à désirer. Enfin, certaines prothèses connues sont sujettes au risque de luxation après implantation sur le patient.

La présente invention a pour but d'éviter ces inconvénients en réalisant une prothèse totale de hanche dont l'implantation soit par ailleurs aisée pour le chirurgien, tout en permettant une adaptation à chaque cas pathologique.

Une prothèse totale de la hanche selon l'invention comprend les caractéristiques de la revendication 1.

Suivant un mode de réalisation préféré de l'invention, le cotyle comporte un dispositif d'indexage constitué d'un ergot disposé à la base du boîtier métallique et d'encoches ménagées à la base de l'insert pour définir parmi plusieurs orientations possibles la position angulaire de l'insert à l'intérieur du boîtier métallique.

Suivant un mode de réalisation préféré de l'invention, le boîtier métallique du premier élément est fixé dans l'os du bassin au moyen d'un ciment.

Suivant un mode de réalisation préféré de l'invention, la partie externe du boîtier métallique du premier élément possède des cônes d'éjection dessinés en creux pour faciliter le passage du ciment.

Suivant un mode de réalisation préféré de l'invention, le boîtier métallique du premier élement est fixé dans l'os au moyen de vis engagées dans des trous répartis sur la surface dudit boîtier.

Suivant un mode de réalisation préféré de l'invention, la paroi interne des trous présente une forme convergente qui définit une portée intérieure en forme de calotte sphérique.

Suivant un mode de réalisation préféré de l'invention, l'insert est en polyéthylène.

Le dessin annexé donné à titre d'exemple non limitatif permettra de mieux comprendre les caractéristiques de l'invention et les avantages qu'elle est susceptible de lui procurer.
- la figure 1 est une vue en perspective montrant le boîtier métallique du premier élément, dans le cas d'une fixation sans ciment, au moyen de vis.
- la figure 2 est une vue en plan d'une variante, montrant les huit encoches périphériques pour le positionnement angulaire, d'un insert en polyéthylène.
- la figure 3 est une vue en coupe diamétrale de l'élément de la figure 1.
- la figure 4 montre à grande échelle un détail correspondant à la structure de revêtement poreuse.
- la figure 5 est une coupe diamétrale d'un mode de réalisation de l'insert en polyéthylène.
- la figure 6 est une coupe correspondant à un détail de la figure 1 et montrant la forme sphérique de l'appui d'une tête de vis.
- la figure 7 est un schéma d'ensemble montrant la prothèse selon l'invention après assemblage.
- la figure 8 est une vue de face de la pièce fémorale dépourvue de sa sphère d'articulation.
- la figure 9 en est une vue latérale.
- la figure 10 montre une variante où la zone proximale évasée est revêtue d'une structure poreuse.
- la figure 11 est une coupe montrant cette partie de la pièce fémorale après son implantation dans le fémur.
- la figure 12 est une vue éclatée montrant l'insert et le boîtier métallique avant leur encliquetage l'un dans l'autre.
- la figure 13 est une vue latérale du boîtier.
- la figure 14 en est une coupe diamétrale à plus grande échelle.
- la figure 15 montre sous un angle différent le boîtier et l'insert avant leur assemblage.
- la figure 16 est une vue en plan de la face inférieure d'un boîtier métallique, dans sa variante à fixation vissée.
- la figure 17 montre la tête à appui sphérique d'une des vis de fixation de ce boîtier.
- la figure 18 est une coupe diamétrale de l'insert, dans une variante comportant une "visière" antiluxation.

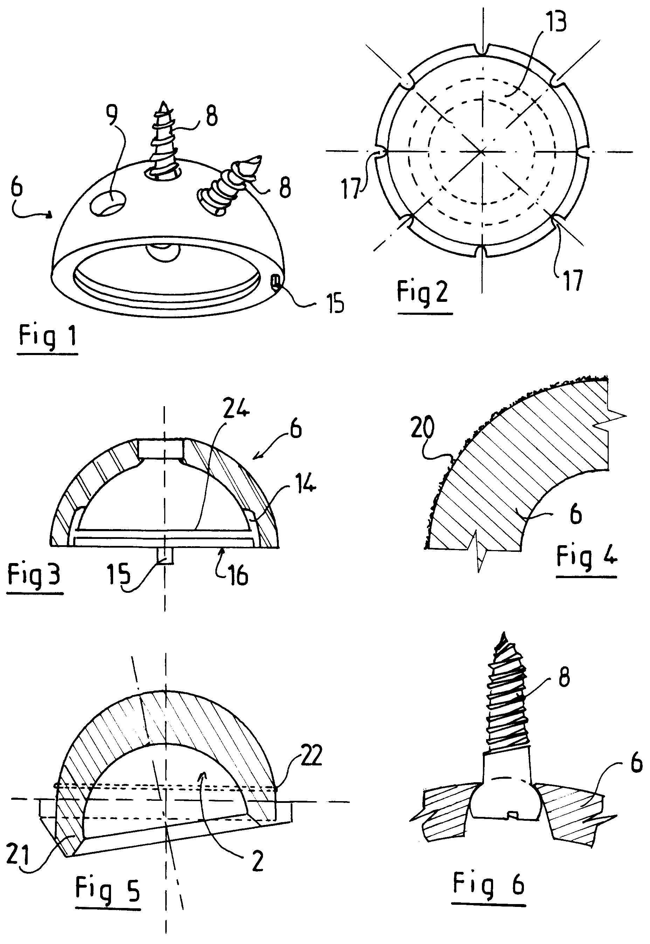

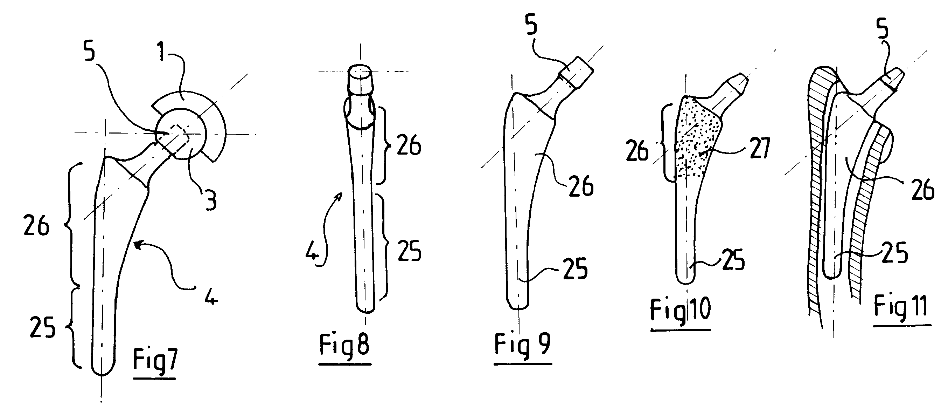

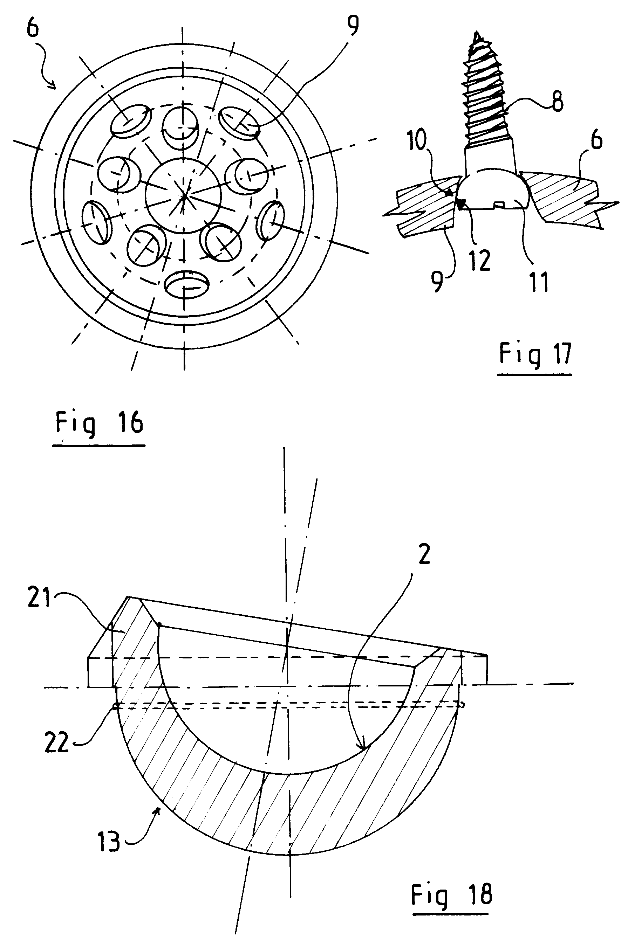

On a représenté sur les figure 1 à 7 et 12 à 18 le premier élément "cotyle" 1 une prothèse totale de la hanche selon l'invention. Ce premier élément 1 possède en creux un logement 2 en forme de calotte sphérique, destiné à venir s adapter sur la rotule 3 (figure 7) d'un deuxième élément ou "pièce fémorale 4". Dans l'exemple choisi, on a supposé que la pièce fémorale 4 se termine à sa partie supérieure par une portée tronconique 5 sur laquelle vient s'emboîter la rotule sphérique 3, ceci suivant une technique bien connue.

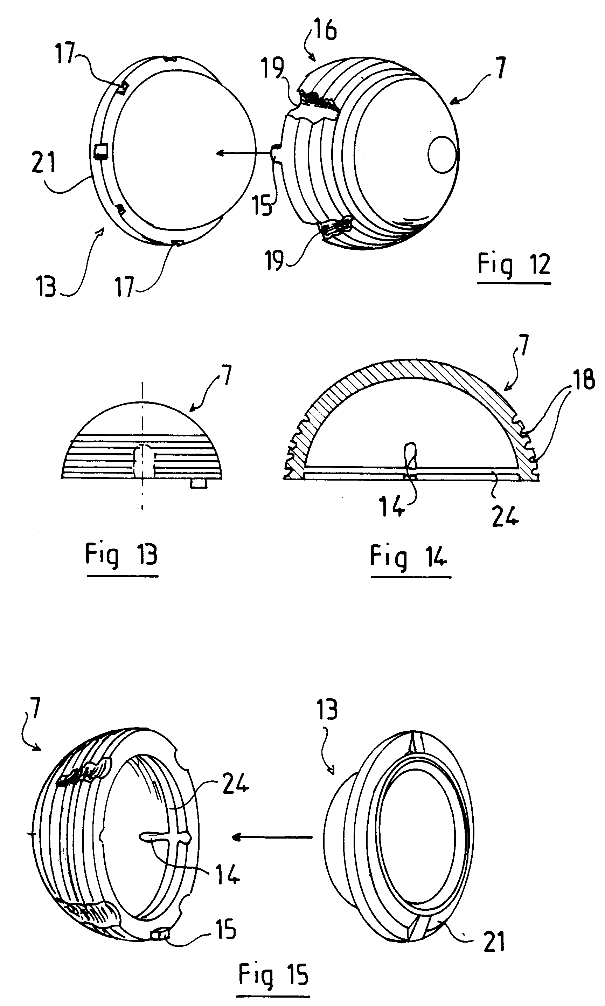

Selon l'invention, le premier élément 1 est réalisé en deux pièces, à savoir un boîtier extérieur métallique 6 (figures 1, 6, 16, 17) ou 7 (figures 12 à 15).

En ce qui concerne le boîtier métallique 6, son implantation s'effectue par vissage à l'aide de vis 8 que le chirurgien met en place dans l'os du bassin du patient. Pour cela, on prévoit plusieurs trous 9 répartis à différentes positions angulaires tout autour de la calotte sphérique formant le boîtier 6. En fonction de l'état du patient, le chirurgien choisit plusieurs de ces trous 9 pour y engager des vis 8 (par exemple seulement au nombre de trois ou quatre. Pour faciliter encore cette implantation et notamment le choix de l'inclinaison de chaque vis 8 dans son trou 9, la paroi interne de ce dernier présente une forme convergente qui définit une portée intérieure 10 en forme de calotte sphérique. Sur cette portée vient prendre appui la face périphérique de la tête 11 de la vis 8, face périphérique 12 qui possède elle aussi une paroi en forme de calotte sphérique. Ainsi, on comprend que le chirurgien ait toute facilité pour incliner la vis 8 dans une direction ou dans l'autre, tandis que sa tête 11 conserve une bonne portée dans le trou 9.

L'insert 13 à encliqueter dans le boîtier métallique 6 est réalisé en une matière synthétique telle que par exemple de polyéthylène. Son encliquetage dans le boîtier 6 est facilité par des cônes d'éjection d'air 14 prévus en creux dans la face interne de celui-ci. Par ailleurs, un dispositif d'indexage est prévu pour définir la position angulaire de l'insert 13 dans le boîtier métallique 6. Ce dispositif d'indexage peut comporter un ergot 15 dépassant hors de la face d'appui 16 du boîtier 6, pour permettre de l'engager dans l'une ou l'autre des encoches 17 prévues en regard autour de l'insert 13.

Dans la variante des figures 12 à 15, la fixation du boîtier 7 dans l'os du bassin du patient s'effectue, non plus par des vis, mais à l'aide d'un ciment. Pour cela, on prévoit sur la paroi extérieure du boîtier 7 :
- d'une part des rainures périphériques 18 facilitant l'accrochage du ciment ;
- d'autre part des cônes d'éjection 19 dessinés en creux sur l'extérieur du boîtier 7, pour que le ciment encore liquide puisse baver au moment de l'implantation dans l'os.

Dans tous les cas, que le boîtier 6 ou 7 soit vissé ou cimenté, il est avantageux de revêtir sa surface extérieure au moyen d'une structure poreuse 20 (figure 4) destinée à faciliter la fixation, par réhabitation de l'os environnant qui prolifère dans cette structure 20.

L'insert 13 étant ainsi positionné très exactement dans l'os du bassin, il est avantageux de prévoir à ce qui sera sa partie supérieure externe, une partie dépassante ou "visière" 21 susceptible d'empêcher tout déboîtement intempestif de la rotule 3 une fois implantée la prothèse. Cette "visière" 21 est donc appelée à jouer un rôle antiluxation.

Enfin, pour améliorer encore l'assemblage du premier et du deuxième élément, on prévoit avantageusement une nervure périphérique 22 en relief autour de l'insert 13, ce qui permet un encliquetage dans la gorge périphérique interne 24 prévue en regard dans le boîtier 6 ou 7.

En ce qui concerne la pièce fémorale 4 selon l'invention, sa queue présente la particularité d'être définie par deux zones successives 25 et 26, à savoir :
- une zone distale cylindrique 25 prévue pour assurer un bon guidage dans la cavité médullaire du fémur :
- une zone proximale 26, évasée vers le haut dans les quatre directions (figures 7 à 11) pour assurer une bonne répartition des efforts de poussée dus au poids du corps du patient.

Suivant un mode de réalisation préféré, la zone évasée 26 a sa paroi extérieure revêtue d'une structure poreuse 27 facilitant comme précédemment la réhabitation osseuse autour de la prothèse.

## Revendications

1. Prothèse totale de la hanche comprenant :
- d'une part un premier élément ou cotyle (1) à implanter dans le bassin du patient, réalisé en deux pièces, à savoir un boîtier extérieur métallique (6, 7) et un insert (13) en une matière synthétique définissant le logement sphérique (2) de la rotule (3) ;
- d'autre part un deuxième élément ou pièce fémorale (4) à implanter dans la partie supérieure de la cavité médullaire du fémur, comportant une tête supérieure à rotule (3) et une queue d'implantation inférieure dont la forme extérieure définit deux zones successives, à savoir une zone distale cylindrique (25) et une zone proximale (26) évasée ; caractérisée en ce que le boîtier métallique (6, 7) du premier élément (1) possède des cônes d'éjection d'air (14) ménagés en creux dans sa face interne pour faciliter l'encliquetage de l'insert (13) en polyéthylène dans le boîtier métallique (6, 7), et en ce que la zone proximale (26) de la pièce fémorale (4) est évasée vers le haut dans les quatre directions.

2. Prothèse suivant la revendication 1, caractérisée en ce que le cotyle (1) comporte un dispositif d'indexage constitué d'un ergot (15) disposé à la base du boîtier métallique (6) et d'encoches (17) ménagées à la base de l'insert (13) pour définir parmi plusieurs orientations possibles la position angulaire de l'insert (13) à l'intérieur du boîtier métallique (6, 7).

3. Prothèse suivant la revendication 1, caractérisée en ce que le boîtier métallique (7) du premier élément (1) est fixé dans l'os du bassin au moyen d'un ciment.

4. Prothèse suivant la revendication 3, caractérisée en ce que la partie externe du boîtier métallique (6) du premier élément (1) possède des cônes d'éjection (19) dessinés en creux pour faciliter le passage du ciment.

5. Prothèse suivant la revendication 1, caractérisée en ce que le boîtier métallique (6) du premier élement (1) est fixé dans l'os au moyen de vis (8) engagées dans des trous (9) répartis sur la surface dudit boitier (6).

6. Prothèse suivant la revendication 5, caractérisée en ce que la paroi interne des trous (9) présente une forme convergente qui définit une portée intérieure (10) en forme de calotte sphérique.

7. Prothèse suivant l'une quelconque des revendications précédentes, caractérisée en ce que l'insert (13) est en polyéthylène.

## Claims

1. Total hip-replacement prosthesis comprising:
- on the one hand a first element or acetabulum (1) to be implanted in the pelvis of the patient, which is made in two parts, namely a metal outer casing (6, 7) and an insert (13) made of a synthetic material defining the spherical recess (2) of the ball and socket joint (3);
- on the other hand a second element or femoral part (4) to be implanted in the upper part of the medullar cavity of the femur, comprising an upper ball-shaped head (3) and a lower implanting tail the outer shape of which defines two successive zones, namely a cylindrical distal zone (25) and a widened proximal zone (26); characterised in that the metal casing (6, 7) of the first element (1) has air ejection tapers (14) recessed in its inner surface to facilitate the engagement of the polyethylene insert (13) in the metal casing (6, 7), and in that the proximal zone (26) of the femoral part (4) widens upwardly in all four directions.

2. Prosthesis according to claim 1, characterised in that the acetabulum (1) comprises an indexing device consisting of a lug (15) arranged on the base of the metal casing (6) and notches (17) provided on the base of the insert (13) to define, among numerous possible orientations, the angular position of the insert (13) inside the metal casing (6, 7).

3. Prosthesis according to claim 1, characterised in that the metal casing (7) of the first element (1) is fixed in the bone of the pelvis by means of a cement.

4. Prosthesis according to claim 3, characterised in that the outer part of the metal casing (6) of the first element (1) has ejection tapers (19) hollowed out to facilitate the insertion of the cement.

5. Prosthesis according to claim 1, characterised in that the metal casing (6) of the first element (1) is fixed in the bone by means of screws (8) engaging in holes (9) distributed over the surface of said casing (6).

6. Prosthesis according to claim 5, characterised in that the inner wall of the holes (9) has a convergent shape which defines an inner span (10) in the shape at a spherical cup.

7. Prosthesis according to any one of the preceding claims, characterised in that the insert (13) consists of polyethylene.

## Patentansprüche

1. Hüftgelenkprothese, welche folgendes aufweist:
- einerseits ein erstes Element oder Kotyl (1), welches in das Becken des Patienten implantiert wird und aus zwei Teilen besteht, das heißt, einem äußeren metallischen Gehäuse (6, 7) und einem Einsatz (13) aus einem Kunststoff, welcher die kugelförmige Aufnahme (2) des Kugelgelenkes (3) bildet;
- andererseits ein zweites Element oder einen femoralen Teil (4), welcher in den oberen Teil des medullären Hohlraumes des Schenkelhalsknochens implantiert wird und einen oberen kugelförmigen Kopf (3) und einen unteren Implantationsabschnitt aufweist, dessen äußere Form aus zwei aufeinanderfolgenden Zonen besteht, das heißt, einer distalen zylindrischen Zone (25) und einer aufgeweiteten proximalen Zone (26);
**dadurch gekennzeichnet**, **daß**
das metallische Gehäuse (6, 7) des ersten Elementes (1) konische Entlüftungsöffnungen (14) aufweist, welche in seiner Innenfläche vorgesehen sind, um das Einklinken des Einsatzes (13) aus Polyethylen in das metallische Gehäuse (6, 7) zu erleichtern, und dadurch, daß die proximale Zone (26) des femoralen Teils (4) nach oben in allen vier Richtungen aufgeweitet ist.

2. Hüftgelenkprothese nach Anspruch 1,
**dadurch gekennzeichnet**, **daß**
das Kotyl (1) eine Indexierung aufweist, welche aus einer Nase (15), die an der Unterseite des metallischen Gehäuses (6) angebracht ist, sowie aus Kerben (17) besteht, die an der Unterseite des Einsatzes (13) vorgesehen sind, um unter mehreren Möglichkeiten die jeweilige Winkelposition des Einsatzes (13) innerhalb des metallischen Gehäuses (6, 7) auszuwählen.

3. Hüftgelenkprothese nach Anspruch 1,
**dadurch gekennzeichnet**, **daß**
das metallische Gehäuse (7) des ersten Elementes (7) in den Beckenknochen einzementiert wird.

4. Hüftgelenkprothese nach Anspruch 3,
**dadurch gekennzeichnet**, **daß**
die Außenwand des metallischen Gehäuses (6) des ersten Elementes (1) Austrittsöffnungen (19) aufweist, um den Durchgang des Zementes zu erleichtern.

5. Hüftgelenkprothese nach Anspruch 1,
**dadurch gekennzeichnet**, **daß**
das metallische Gehäuse (6) des ersten Elementes (1) im Knochen mit Hilfe einer Schraube (8) befestigt wird, welche in Bohrungen (9) eingreift, die über die Oberfläche des Gehäuses (6) verteilt angeordnet sind.

6. Hüftgelenkprothese nach Anspruch 5,
**dadurch gekennzeichnet**, **daß**
die Innenwand der Bohrungen (9) eine konvergierende Form besitzt, welche eine innere Auflagefläche (10) in Form einer kugelförmigen Kalotte bildet.

7. Hüftgelenkprothese nach einem der vorausgegangenen Ansprüche,
**dadurch gekennzeichnet**, **daß**
der Einsatz (13) aus Polyethylen besteht.
